# EUROPEAN PATENT APPLICATION

(11) **EP 4 803 536 A1**
(43) Date of publication of application: **09.09.2026**
(21) Application number: 25161991.2
(22) Date of filing: 06.03.2025
(51) Int. Cl.: C07K 14/71, A61K 38/16

(54) **NOVEL FUSIONS PROTEINS FOR EXTENDED HALF-LIFE OF VEGF BINDING PROTEINS**

(71) Applicant: NAVIGO PROTEINS GMBH, 06120 Halle/Saale (DE)
(72) Inventor: HAUPTS, Ulrich, 06120 Halle/Saale (DE); COBURGER, Ina, 06120 Halle/Saale (DE); HOFFMANN, Gregor, 06120 Halle/Saale (DE); BOSSE-DOENECKE, Eva, 06120 Halle/Saale (DE)
(74) Representative: Haucke, Kerstin

(57) **Abstract**

The present invention relates to novel fusion proteins with prolonged half-life of VEGF binding proteins. The invention refers to novel engineered fusion proteins for use in anti-angiogenic therapy, in particular for treating angiogenesis-related diseases and disorders of the eye. The fusion proteins comprise a subunit that specifically binds to serum albumin. In addition to the serum albumin binding protein, the new fusion proteins comprise a protein therapeutically effective in treating diseases of the eye, such as Aflibercept. The fusion proteins are particularly well-suited for medical applications that require extended half-life of eye specific therapeutic proteins.

## Description

### FIELD OF THE INVENTION

The present invention relates to novel fusion proteins with prolonged half-life of VEGF binding proteins. The invention refers to novel engineered fusion proteins for use in anti-angiogenic therapy, in particular for treating angiogenesis-related diseases and disorders of the eye. The fusion proteins comprise a subunit that specifically binds to serum albumin. In addition to the serum albumin binding protein, the new fusion proteins comprise a protein therapeutically effective in treating diseases of the eye, such as Aflibercept. The fusion proteins are particularly well-suited for medical applications that require extended half-life of eye specific therapeutic proteins.

### BACKGROUND OF THE INVENTION

Disorders of the eye are common diseases affecting many individuals. Neovascular eye diseases such as for example neovascular (wet) age-related macular degeneration (AMD) are the leading cause of irreversible vision loss among the aging population and affect more than 4 million individuals in the developed countries. AMD causes damage to the macula, which is a spot centrally located on the retina of the eye and results in loss of sharp, central vision. Other retinal neovascular diseases include diabetic macular edema (DME), diabetic retinopathy (DR), and macular edema following retinal vein occlusion (RVO). In diabetic retinopathy (DR), a common complication in diabetes, damaged blood vessels of the light-sensitive tissue at the back of usually both eyes are the reason for impaired vision or even vision loss. After the development of diabetic retinopathy patients may be affected by diabetic macular edema where fluids acculumulate in the macula of the retina due to leaking blood vessels. Retinal vein occlusion is an eye disease where veins in the retina are blocked resulting in blurry vision or sudden blindness.

Current procedures for the treatment of eye diseases such as AMD include VEGF-binding proteins such Aflibercept, Conbercept, Ranibizumab, Bevacizumab, or biosimilars of VEGF binding proteins. Although a topical application of these drugs is possible, a disadvantage of the topical application is a very fast wash out from the eye and accordingly, a very low fraction of drug uptake. An alternative to topical applications to the eye are injections; however, the treatment of eye diseases requires frequent intraocular injections of drugs as often as every 1-2 months, into one or both eyes, often for life. This sums up to 6-12 injections per year. Pharmaceutical interventions through injections into the vitreous are inconvenient for patients since they are risky and painful for the patients.

Thus, needless to say that existing therapies for eye diseases have significant disadvantages for patients. They are burdensome for patients in terms of pain, cost, time, and risk. There is a strong need to relief patients from the imperative frequent and burdening treatments of eye diseases.

Due to significant limitations of current therapies for eye diseases, there was a need to provide novel proteins for the treatment of ocular diseases with improved properties, in particular in view of prolonged availability in the eye to reduce the painful frequent injection. Accordingly, there is a need in this field to obtain novel proteins suitable for more effective approaches for the therapy of eye diseases.

One objective of the present invention is the provision of molecules for improving the half life of a therapeutic protein effective for eye diseases to prolong the beneficial impact of the therapeutic protein such as Aflibercept for eye diseases.

The present invention provides artificial fusion proteins for a therapeutic protein effective for eye diseases and serum albumin binding proteins that are particularly well-suited for the treatment of eye diseases but overcome the disadvantages of current approaches.

The above-described objectives and advantages are achieved by the subject-matters of the enclosed claims. The present invention meets the needs presented above by providing examples for fusion proteins. Preferred embodiments of the invention are included in the claims as well as in the following description, examples and figures. The above overview does not necessarily describe all problems solved by the present invention.

### SUMMARY OF THE INVENTION

The present invention provides novel fusion proteins with prolonged half-life of VEGF binding proteins. The novel fusion proteins comprise a subunit that specifically binds to serum albumin. The fusion proteins are particularly well-suited for medical applications that require extended half-life of eye specific therapeutic proteins. Accordibngly, the invention provides novel engineered fusion proteins for use in anti-angiogenic therapy, in particular for treating angiogenesis-related diseases and disorders of the eye.

The present disclosure provides the following items 1 to 15, without being specifically limited thereto.

In particular, the present invention provides:
[1] A fusion protein capable of binding to VEGF and serum albumin, comprising:
   a VEGF binding protein comprising ligand-binding elements from the extracellular components of VEGF receptor (VEGFR)-1 and/or VEGF receptor (VEGFR)-2; and
   a serum albumin binding protein comprising the amino acid sequence of SEQ ID NO: 1, or an amino acid sequence with at least 90% sequence identity to SEQ ID NO: 1.
[2] The fusion protein of [1], wherein the ligand-binding elements from the extracellular components of VEGFR-1 and/or VEGFR-2 are fused to the Fc portion of an immunoglobulin, preferably to the Fc portion of an IgG.
[3] The fusion protein of [1] or [2], wherein the ligand-binding elements from the extracellular components of VEGFR-1 and VEGFR-2 comprise the second (Ig) domain of VEGFR-1 and the third (Ig) domain of VEGFR-2, respectively.
[4] The fusion protein of any one of [1] to [3], wherein the VEGF binding protein comprises an amino acid sequence with at least 90 %, preferably at least 95 %, sequence identity to SEQ ID NO: 3, and wherein the fusion protein exhibits binding affinity for VEGF, preferably wherein the VEGF binding protein is Aflibercept or a biosimilar of Aflibercept.
[5] The fusion protein of any one of [1] to [4], wherein the serum albumin binding protein has a binding affinity to human, cynomolgus, rat, and/or mouse serum albumin, of less than 10 nM, preferably of less than 1 nM.
[6] The fusion protein of any one of [1] to [5], wherein the serum albumin binding protein comprises the amino acid sequence of SEQ ID NO: 2, or an amino acid sequence with at least 90% sequence identity to SEQ ID NO: 2, and/or wherein the VEGF binding protein has a binding affinity to VEGF of less than 10 nM, preferably of less than 1 nM.
[7] The fusion protein of any one of [1] to [6], wherein the VEGF binding protein and the serum albumin binding protein are linked via a linker, preferably wherein the linker is a peptide linker.
[8] The fusion protein of any one of [1] to [7], comprising the amino acid sequence of SEQ ID NO: 4 or 5, or an amino acid sequence with at least 90% sequence identity to SEQ ID NO: 4 or 5.
[9] The fusion protein of any one of [1] to [8], wherein the half-life of the fusion protein is at least 1.5-fold, or at least 2-fold, longer in the vitreous body than the half-life of the VEGF binding protein not fused to serum albumin binding protein.
[10] A pharmaceutical composition comprising the fusion protein of any one of [1] to [9] and a pharmaceutically acceptable carrier and/or diluent.
[11] The fusion protein of any one of [1] to [9], or the pharmaceutical composition of [10], for use in the treatment of eye diseases, preferably for use in the treatment of neovascular eye diseases.
[12] A nucleic acid molecule encoding the fusion protein of any one of [1] to [9].
[13] A vector comprising the nucleic acid molecule of [12].
[14] A host cell, or a non-human host, comprising the fusion protein of any one of [1] to [9], the nucleic acid molecule of [12], and/or the vector of [13].
[15] A method for the production of the fusion protein of any one of [1] to [9], comprising culturing the host cell or the non-human host of [14] under conditions suitable to obtain said fusion protein, and optionally isolating the said fusion protein.

One aspect of the present invention relates to a method for the treatment of an eye disease, the method comprising administering to the eye of a subject in need thereof a therapeutically effective amount of the fusion protein of any one of items [1] to [9], or the pharmaceutical composition of item [10], in an amount and via a route sufficient to treat the eye disease.

This summary of the invention does not necessarily describe all features of the present invention. Other embodiments become apparent from a review of the ensuing detailed description.

### BRIEF DESCRIPTION OF THE FIGURES.

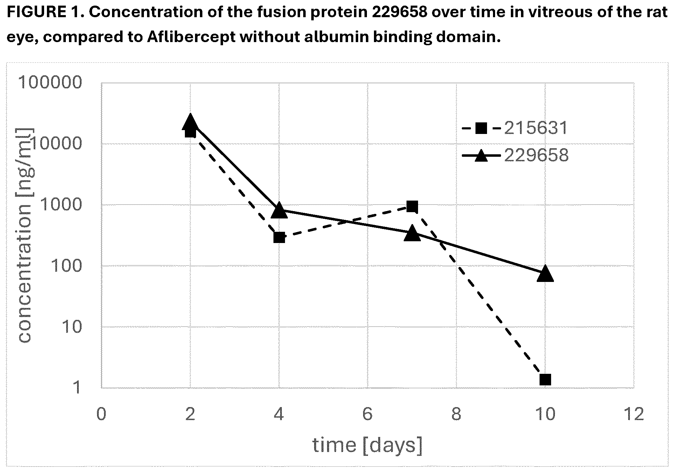

**Figure 1** shows that fusion protein 229658 binds to serum albumin in the vitreous of rat eye. Fusion protein 229658 or Aflibercept were injected into the vitreous of rat eye; rats were terminated at different time points and eyes extracted. Shown are the concentrations (ng/ml) of different time points up to 10 days of fusion protein 229658 (Aflibercept with albumin binding domain; black triangle and solid line) and Aflibercept (without albumin binding domain; 215631; black square) by ELISA. The lines represent best fits to a four-parameter logistic binding model. The concentration of aflibercept is 76 ng/ml after 10 days, whereas the concentration of the fusion protein is 1.4 ng/ml after 10 days. The concentration of the fusion protein is about 54-fold higher after 10 days than Aflibercept.

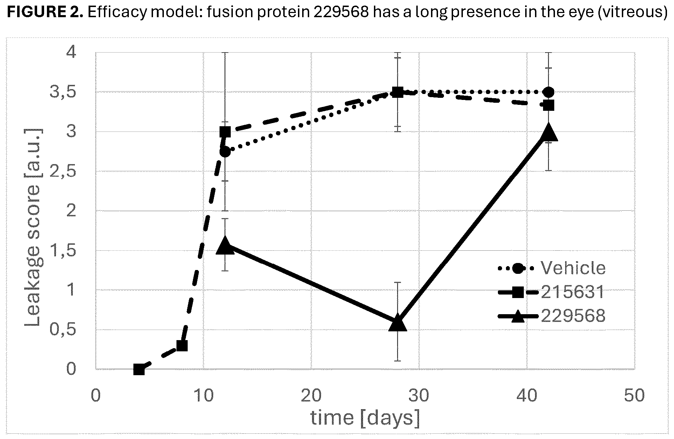

**Figure 2** shows an efficacy study. Measured was the leakage score of the fusion protein 229658 (filled triangle, solid line) compared to Aflibercept (215631, solid square) and empty vehicle (filled dot). A low score over a long time is indicative of a long presence of the fusion protein 229568 in the vitreous of the eye. Figure 2 shows that the fusion protein has a long presence of at least about 30 days in the vitreous of the eye.

### DETAILED DESCRIPTION OF THE INVENTION

The novel fusion proteins provided by the present invention provide for a prolonged half-life of VEGF binding proteins. The novel fusion proteins are based, at least in part, on a subunit that specifically binds to serum albumin. Accordingly, as part of the present invention, provided herein are novel serum albumin binding proteins comprising the amino acid sequence of SEQ ID NO: 1 or SEQ ID NO: 2, or an amino acid sequence with at least 90% sequence identity to SEQ ID NO: 1 or SEQ ID NO: 2, respectively. The novel serum albumin binding proteins show a binding affinity to human, cynomolgus, rat, and/or mouse serum albumin. The novel serum albumin binding proteins exhibit a binding affinity to human, cynomolgus, rat, and/or mouse serum albumin, of less than 10 nM, preferably of less than 1 nM. In preferred embodiments, the novel serum albumin binding proteins exhibit a binding affinity to human and mouse serum albumin of less than 10 nM, preferably of less than 1 nM. In particularly preferred embodiments, the novel serum albumin binding proteins exhibit a binding affinity to human and mouse serum albumin of less than 0.5 nM.

The novel serum albumin binding proteins are particularly well-suited for use in medical applications that require extended half-life of eye specific therapeutic proteins. Specifically, the novel serum albumin binding proteins are particularly well-suited for use in fusion proteins comprising a protein capable of binding to VEGF as described in the present invention. Such proteins capable of binding to VEGF are preferably VEGF binding proteins comprising ligand-binding elements from the extracellular components of VEGF receptor (VEGFR)-1 and/or VEGF receptor (VEGFR)-2, as described elsewhere herein.

The fusion proteins of the invention bind to serum albumin. The novel serum albumin binding proteins and the novel fusion proteins of the invention bind to the major component of the vitreous body of the eye, serum albumin. The VEGF-binding part of the novel fusion proteins of the invention is a therapeutic protein effective in the therapy of eye diseases, including treatment and prevention of eye diseases. Due to the anchoring of the fusion protein in the eye by binding to serum albumin, the local residence time of the therapeutic protein is significantly enhanced so that much less frequent painful treatments of eye diseases are required. The novel fusion proteins of the invention enable fewer medical interventions and safer therapies in eye diseases and improve quality of life for patients.

The present inventors have developed a solution to meet the ongoing need in the art by providing fusion proteins comprising serum albumin specific proteins and a therapeutic protein effective in anti-angiogenic therapy, in particular for the treatment of angiogenesis-related eye diseases. The fusion proteins are functionally characterized, *inter alia,* by a high affinity for serum albumin. The fusion proteins of the invention provide molecular formats with favorable physicochemical properties, in particular, they are stable and may broaden therapeutic options. Therapeutic proteins for eye diseases, if fused to the novel serum albumin binding proteins as disclosed herein, may have a longer duration of action in eye diseases since the clearance of the therapeutic protein from the vitreous body is decreased and the half-life is extended. The novel fusion proteins provided herein, and the related compositions and methods, allow for retention of therapeutic proteins for eye diseases for a longer period of time. Further, this enhances patient acceptance and quality of life and is an improvement over current treatment strategies.

Before the present invention is described in more detail below, it is to be understood that this invention is not limited to the particular methodology, protocols and reagents described herein as these may vary. It is also to be understood that the terminology used herein is for the purpose of describing particular aspects and embodiments only and is not intended to limit the scope of the present invention, which is reflected by the appended claims. Unless defined otherwise, all technical and scientific terms used herein have the same meaning as commonly understood by one of ordinary skill in the art to which this invention belongs. This includes a skilled person working in the field of protein engineering and purification, but also including a skilled person working in the field of developing new fusion molecules for use in anti-angiogenic therapy, in particular in the treatment of various eye diseases, specifically angiogenesis-related eye diseases. In particularly preferred embodiments, the anti-angiogenic therapy is an anti-VEGF therapy, and the treatment of various eye diseases is specifically the treatment of VEGF-related eye diseases.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

Throughout this specification and the claims, which follow, unless the context requires otherwise, the word "comprise", and variants such as "comprises" and "comprising", was understood to imply the inclusion of a stated integer or step, or group of integers or steps, but not the exclusion of any other integer or step or group of integers or steps. The term "comprise(s)" or "comprising" may encompass a limitation to "consists of" or "consisting of", should such a limitation be necessary for any reason and to any extent.

Several documents (for example: patents, patent applications, scientific publications, manufacturer's specifications, instructions, GenBank Accession Number sequence submissions etc.) may be cited throughout the present specification. Nothing herein is to be construed as an admission that the invention is not entitled to antedate such disclosure by virtue of prior invention.

All sequences referred to herein are disclosed in the attached sequence listing that, with its whole content and disclosure, forms part of the disclosure content of the present specification.

### GENERAL DEFINITIONS OF TERMS USED IN THE APPLICATION

The terms "serum albumin binding protein" refers to a protein capable of binding to serum albumin.

The term "fusion protein" relates to a protein comprising at least a first protein joined genetically to at least a second protein. A fusion protein is typically created through joining of two or more genes that originally code for separate proteins. Fusion proteins may further comprise additional domains that are not involved in binding of the target(s), such as but not limited to, for example, multimerization moieties, polypeptide tags, polypeptide linkers, half-life extending moieties.

The terms "protein" and "polypeptide" refer to any chain of two or more amino acids linked by peptide bonds and does not refer to a specific length of the product. Thus, "peptides", "protein", "amino acid chain", or any other term used to refer to a chain of two or more amino acids, are included within the definition of "protein", and the term "protein" may be used instead of, or interchangeably with, any of these terms. The term "protein" is also intended to refer to the products of post-translational modifications of the polypeptide which are well known in the art. The terms "protein" and "polypeptide" also include antibodies or antibody fragments. The term "antibody" refers to an immunoglobulin molecule or immunoglobulin-derived molecule that specifically binds to, or is immunologically reactive with, an antigen or epitope, and includes both polyclonal and monoclonal antibodies, as well as functional antibody fragments, including but not limited to fragment antigen-binding (Fab) fragments, F(ab')2 fragments, Fv fragments, recombinant IgG (rlgG) fragments, single chain variable fragments (scFv), and single domain antibodies (e.g., sdAb, sdFv, nanobody, VHH fragments). The antibody may thus be a single domain antibody or comprise at least one variable light and at least one variable heavy chain. In one embodiment, the at least one variable light and at least one variable heavy chain are displayed as a single polypeptide chain. In certain embodiments, the term "antibody" or "antigen binding protein" includes germline derived antibodies. The term "antibody" or "antigen binding protein" includes genetically engineered or otherwise modified forms of immunoglobulins, such as intrabodies, peptibodies, chimeric antibodies, fully human antibodies, humanized antibodies, *etc.*

The term "decoy protein" as used herein typically means a decoy receptor that is capable to bind a growth factor. In the present invention, such growth factors include Vascular Endothelial Growth Factor (VEGF) and Placenta (or Placental) Growth Facor (PLGF). As described herein, a "decoy protein" (or "decoy receptor") does not require mimicking the entire native receptor of a growth factor, but may comprise parts thereof. For example, a preferred VEGF binding protein of the present invention is a protein comprising ligand-binding elements from the extracellular components of VEGF receptor (VEGFR)-1 and/or VEGF receptor (VEGFR)-2. The ligand-binding elements from the extracellular components of VEGFR-1 and VEGFR-2 may comprise the second (Ig) domain of VEGFR-1 and the third (Ig) domain of VEGFR-2, respectively. Such a VEGF binding protein can be considered a "decoy protein" or "decoy receptor" of the present invention.

As decribed elsewhere herein, the ligand-binding elements from the extracellular components of VEGFR-1 and/or VEGFR-2 may be fused to the Fc portion of an immunoglobulin, preferably to the Fc portion of an IgG. Particualrly preferred is the Fc portion of IgG1.

In the present invention, a "decoy protein" or "decoy receptor" comprises an amino acid sequence with at least 90 %, preferably at least 95 %, sequence identity to SEQ ID NO: 3, and exhibits binding affinity for VEGF. Aflibercept or a biosimilar of Aflibercept can be considered as "decoy protein" or "decoy receptor" of the present invention.

The terms "therapeutic protein for eye diseases" or "protein therapeutically effective in eye diseases" may be interchangeably and relate to a protein that is used for therapies of eye diseases. Thus, a therapeutic protein for eye diseases is understood as a protein for use in treating a disorder that affects the eye. A therapeutic protein effective in the treatment of eye diseases may be used for the treatment or prevention of neovascular (wet) age-related macular degeneration (AMD), diabetic macular edema (DME), diabetic retinopathy (DR), macular edema following retinal vein occlusion (RVO), non-infectious uveitis, thyroid eye disease, neurotrophic keratitits, ocular surface squamous neoplasia, dry eye disease, proliferative vitreoretinopathy, retinoblastoma, atopic keratoconjunctivitis, vitreomacular adhesion, myopic choroidal neovascularization, and other.

The term "VEGF" or "vascular endothelial growth factor" is a human vascular endothelial growth factor. VEGF-A (uniprot Accession Number P15692) exists as a number of different isotypes which are generated both by alternative splicing and proteolysis, for example, VEGF-206, VEGF-189, VEGF-165, and VEGF-121. The isoforms are all biologically active as dimers. VEGF herein means any of the natural isoforms or natural variants or induced variants having at least a sequence identity of at least 80 %, 85 %, 90 %, 95 %, 96 % or 97 % or more, or 100 % to a natural isoform or natural variant. The terms "protein with binding specificity for VEGF", "VEGF binding protein", and "VEGF specific binding protein" may be used interchangeably herein and refer to a protein with high affinity binding to VEGF. The VEGF binding protein used in the present invention exhibits binding affinity specifically for VEGF-A. The VEGF binding protein used in the present invention may also be capable of binding to VEGF-B and/or PLGF. In the present invention, a "VEGF binding protein" is capable of specifically binding to VEGF, in particular VEGF-A. In the present invention, a "VEGF binding protein" may also be capable of specifically binding to VEGF-B and/or PLGF. A preferred "VEGF binding protein" capable of specifically binding to VEGF, in particular VEGF-A, is a VEGF binding protein comprising ligand-binding elements from the extracellular components of VEGF receptor (VEGFR)-1 and/or VEGF receptor (VEGFR)-2. Accordingly, a preferred "VEGF binding protein" capable of specifically binding to VEGF, in particular VEGF-A, includes a decoy protein described elsewhere herein.

In the present invention, a "VEGF binding protein" is not only capable of (specifically) binding to VEGF, in particular VEGF-A, but is also capable of (specifically) inhibiting VEGF, in particular VEGF-A. In the present invention, a "VEGF binding protein" may also be capable of (specifically) inhibiting VEGF-B and/or PLGF. The terms "(specifically) inhibiting VEGF" and "(specifically) blocking VEGF" (including VEGF-A and VEGF-B) may be used interchangeably herein. Likewise, the terms "(specifically) inhibiting PLGF" and "(specifically) blocking PLGF" may be used interchangeably herein.

The term "(specifically) inhibiting VEGF" (including VEGF-A and VEGF-B) includes inhibiting VEGF-mediated signaling, in particular VEGF-A-mediated signaling. Likewise, the term "(specifically) inhibiting PLGF" includes inhibiting PLGF-mediated signaling.

Accordingly, a "VEGF binding protein" of the presnt invention is specifically suitable for inhibiting the interaction of VEGF, in particular VEGF-A, with its native receptors, in particular VEGFR-1 and VEGFR-2. Accordingly, a "VEGF binding protein" of the presnt invention is specifically suitable for decreasing (reducing) or inhibiting VEGF activation of its native receptors, in particular VEGF-A activation of its native receptors, in parrticualr VEGFR-1 and VEGFR-2.

The novel fusion proteins of the present invention show binding affinity for VEGF-A and human and mouse serum albumin of less than 15 nM or less than 10 nM, in particular even less than 1 nM. In preferred embodiments, the novel fusion proteins of the present invention show binding affinity for VEGF-A and human and mouse serum albumin of less than 0.5 nM, as exemplified by the novel fusion protein comprising SEQ ID NO: 3 and SEQ ID NO: 2 (the fusion protein is referred to as 229658 herein).

The term "modification" or "amino acid modification" refers to a substitution, a deletion, or an insertion of an amino acid at a particular position in a parent polypeptide sequence by another amino acid. Given the known genetic code and recombinant and synthetic DNA techniques, the skilled scientist can readily construct DNAs encoding the amino acid variants.

The term "substitution" is understood as exchange of an amino acidby another amino acid. The term "insertion" comprises the addition of amino acids to the original amino acid sequence. The term "binding affinity" refers to the ability of a polypeptide to bind to another polysaccharide, protein, peptide, or fragment or domain thereof. As used herein, binding affinity is typically measured and reported by the equilibrium dissociation constant (K_{D}), which is used to evaluate and rank order strengths of bimolecular interactions.

The term "amino acid sequence identity" refers to a quantitative comparison of the identity (or differences) of the amino acid sequences of two or more proteins. "Percent (%) amino acid sequence identity" with respect to a reference polypeptide sequence is defined as the percentage of amino acid residues in a sequence that are identical with the amino acid residues in the reference polypeptide sequence, after aligning the sequences and introducing gaps, if necessary, to achieve the maximum percent sequence identity. To determine the sequence identity, the sequence of a query protein is aligned to the sequence of a reference protein or polypeptide, for example, to the amino acid sequence of the polypeptide of SEQ ID NO: 1. Methods for sequence alignment are well known in the art. For example, the NCBI BLAST similarity program is preferably employed (Camacho et al. 2009, BMC Bioinformatics 10: 421). For multiple alignment analysis, ClustalL is preferably used (Sievers & Higgins, 2021, Methods Mol Biol. 2231: 3-16). Thus, the percentage of an amino acid sequence identity may be calculated using published or commercially available software with an algorithm which conducts comparison using a base sequence (e.g., SEQ ID NO: 1 in the present invention) as a reference sequence. For example, BLAST, FASTA, or GENETYX (manufactured by Software Development Co., Ltd.) may be used, and these may be run with default parameters. Each amino acid of the query sequence that differs from the aligned reference amino acid sequence at a given position is counted as one difference. An insertion or deletion in the query sequence is also counted as one difference. The sum of differences is then related to the length of the aligned reference sequence to yield a percentage of non-identity or identity, respectively.

As used herein, the term "half-life" may be considered as the "biological half-life" or proteins and peptides. The term "half-life" refers to the time that is required for the concentration of a protein or peptide to be reduced or to decrease by one-half (50 %). The expression "biological half-life" is typically considered to refer to the time it takes for the concentration of the substance in question (here: protein or peptide) in blood, serum or plasma to decrease to half of the initial concentration. The biological half-life may be determined according to conventional methods known to a person of skill in the art. For instance, the biological half-life can be determined based on the concentration in serum, plasma or whole blood. In the present invention, the novel fusion protein shows a half-life that is at least 1.5-fold, or at least 2-fold, longer (higher) in the vitreous body than the half-life of the VEGF binding protein not fused to serum albumin binding protein. The terms "longer" and "higher" may be used interchangeably. Accordingly, in the present invention, the term "(biological) half-life preferably refers to the time it takes for the concentration of the fusion protein in the vitreous (body) to decrease to half (50 %) of the initial concentration.

### DETAILED DESCRIPTION OF THE EMBODIMENTS OF THIS INVENTION

The fusion proteins of the invention are capable of binding to VEGF and serum albumin. The fusion protein of the invention comprises [i] a VEGF binding protein; and [ii] a serum albumin binding protein. For example, the fusion proteins of the invention are capable of binding to VEGF and serum albumin, comprising a VEGF specific protein comprising ligand-binding elements from the extracellular components of VEGF receptor (VEGFR)-1 and/or VEGF receptor (VEGFR)-2; and a binding protein for serum albumin comprising the amino acid sequence of SEQ ID NO: 1, or an amino acid sequence with at least 90% sequence identity to SEQ ID NO: 1.

The fusion proteins for example comprise at least two subunits wherein the one subunit is a binding protein for serum albumin with at least 90%, 93 %, preferably at least 95 % sequence identity to SEQ ID NO: 1, and the other subunit is specific for a therapeutic protein for the treatment or prevention of eye diseases, such as a VEGF specific protein comprising ligand-binding elements from the extracellular components of VEGF receptor (VEGFR)-1 and/or VEGF receptor (VEGFR)-2, or an antibody or antibody fragement thereof, as described elsewhere herein.

The VEGF binding protein according to the present invention is a therapeutic protein specifically suitable for the treatement of eye diseases.

Accordingly, in various embodiments of the present invention, the VEGF-binding protein is a protein that inhibits the function of a growth factor or the function of a growth factor receptor. Hence, in various embodiments of the present invention, the therapeutic protein is a growth factor inhibitor, or a growth factor receptor inhibitor. More specifically, in various embodiments of the present invention, the therapeutic protein is an endothelial growth factor inhibitor, or an endothelial growth factor receptor inhibitor. In particularly preferred embodiments, the vascular endothelial growth factor or vascular endothelial growth factor receptor is a vascular endothelial growth factor or vascular endothelial growth factor receptor associated with a disease or disorder of the eye. In the present invention, the VEGF-binding protein is a protein that inhibits the function of a vascular endothelial growth factor (VEGF), such as Aflibercept, biosimilars of Aflibercept, Conbercept, biosimilars of Conbercept, Ranibizumab, Bevacizumab, Brolucizumab, or fragments thereof, or biosimilars thereof.

For example, the VEGF binding protein according to the present invention binds VEGF-A with high affinity. For example, the VEGF binding protein of the invention binds VEGF-A with a binding affinity (K_{D}) of less than 10 nM for VEGF-A, preferably less than 5 nM, more preferably less than 1 nM for VEGF-A.

For example, the VEGF binding protein comprises ligand-binding elements from the extracellular components of VEGF receptor (VEGFR)-1 and/or VEGF receptor (VEGFR)-2. For example, the VEGF binding protein comprising ligand-binding elements from the extracellular components of VEGF receptor (VEGFR)-1 and/or VEGF receptor (VEGFR)-2 is a decoy protein. For example, the VEGF binding protein comprises ligand-binding elements from the extracellular components of VEGF receptor (VEGFR)-1 and/or VEGF receptor (VEGFR)-2, wherein the ligand-binding elements from the extracellular components of VEGFR-1 and/or VEGFR-2 are fused to the Fc portion of an Immunoglobulin, preferably to the Fc portion of an IgG [IgG₁], preferably wherein the ligand-binding elements from the extracellular components of VEGFR-1 and VEGFR-2 comprise the second (Ig) domain of VEGFR-1 and the third (Ig) domain of VEGFR-2. For example, the VEGF binding protein is Aflibercept or Conbercept; or a biosimilar of Aflibercept or Conbercept.

In further embodiments, the VEGF binding protein comprises an amino acid sequence with at least 90 %, preferably at least 95 %, more preferably at least 97 %, sequence identity to SEQ ID NO: 3, and wherein the fusion protein exhibits specific binding affinity for VEGF, in particular for VEGF-A, more preferably wherein the VEGF specific protein is Aflibercept or a biosimilar of Aflibercept.

In some embodiments, the VEGF binding proteins comprises a domain of VEGFR-1 (second domain) and domains of VEGFR-2 (third and fourth domains) regions fused to the Fc portion of human IgG1 immunoglobulin.

In further embodiments, the VEGF binding protein comprises an amino acid sequence with at least 90 %, preferably at least 95 %, more preferably at least 97 %, sequence identity to SEQ ID NO: 8, and wherein the fusion protein exhibits specific binding affinity for VEGF, in particular for VEGF-A, more preferably wherein the VEGF specific protein is Conbercept or a biosimilar of Conbercept.

As further disclosed herein, the VEGF binding protein is a VEGF-binding antibody or VEGF-binding antibody fragment thereof, in particular a VEGF-A-binding antibody or VEGF-A-binding antibody fragment thereof. For example, the VEGF-binding antibody is a VEGF-binding monoclonal antibody, in particular a VEGF-A-binding monoclonal antibody. For example, the VEGF-binding antibody is a (Fab) fragment, F(ab')2 fragment, Fv fragment, recombinant IgG (rlgG) fragment, single chain variable fragment (scFv) or a single domain antibody (e.g., sdAb, sdFv, nanobody, VHH fragments). For example, the VEGF-binding antibody is selected from nay one of Ranibizumab, Bevacizumab, Brolucizumab, or an antigen (VEGF-(A)-binding) fragment thereof. The VEGF-binding antibody may be a VEGF-(A)-binding biosimilar of Ranibizumab, Bevacizumab, Brolucizumab, or an antigen (VEGF-(A)-binding) fragment thereof.

As described elsewhere herein, the serum albumin binding protein of the present invention is capable of binding serum albumin with high affinity. For example, the serum albumin binding protein of the invention binds (human and mouse) serum albumin with a binding affinity (K_{D}) of less than 10 nM, preferably less than 5 nM, more preferably less than 1 nM.

The serum albumin binding protein of the present invention may comprise the amino acid sequence of SEQ ID NO: 1, or an amino acid sequence with at least 90%, at least 93 %, at least 95 %, at least 98 %, or even at least 99 % sequence identity to SEQ ID NO: 1.

In other embodiments, the serum albumin binding protein of the present invention may comprise the amino acid sequence of SEQ ID NO: 2, or an amino acid sequence with at least 90%, at least 93 %, at least 95 %, at least 98 %, or even at least 99 % sequence identity to SEQ ID NO: 2.

In the fusion proteins of the present invention, the serum albumin binding protein may be located at the N-terminus or at the C-terminus of the fusion protein. In preferred embodiments, the serum albumin binding protein is located at the C-terminus of the fusion polypeptide. In various embodiments, the serum albumin binding protein is located C-terminal of the Ig Fc domain (portion) to which the VEGF-binding protein may be fused, as described elsewhere herein and as exemplified by the fusion protein of 229658 or 229657. Accordingly, in various embodiments, the fusion protein of the present invention comprises a VEGF binding protein comprising ligand-binding elements from the extracellular components of VEGF receptor (VEGFR)-1 and/or VEGF receptor (VEGFR)-2; and a serum albumin binding protein comprising the amino acid sequence of SEQ ID NO: 1, or an amino acid sequence with at least 90% sequence identity to SEQ ID NO: 1 as described elsewhere herein, wherein the ligand-binding elements from the extracellular components of VEGFR-1 and/or VEGFR-2 are fused to the Fc portion of an immunoglobulin, preferably to the Fc portion of an IgG, more preferably to the Fc portion of an IgG1, and wherein the serum albumin binding protein is located at the C-terminus of the fusion protein, i.e., C-terminal of the Ig Fc portion (domain). The fusion protein may comprise, or preferably comprises, two serum albumin binding proteins. In such cases, the two serum albumin binding proteins are preferably located at the C-terminus of the fusion protein, i.e., C-terminal of the Ig Fc portion (domain).

Further, the VEGF binding protein and the (two) serum albumin binding protein(s) may be linked, or are preferably linked, by a (peptide) linker, as exemplified by the fusion protein of 229658 or 229657. In preferred embodiments, the linker has the sequence of SEQ ID NO: 6. Likewise, the two serum albumin binding proteins may be linked, or are preferably linked, by a (peptide) linker, as exemplified by the fusion protein of 229658. In preferred embodiments, the linker connecting the two serum albumin binding proteins has the sequence of SEQ ID NO: 7. Still further, as described elsewhere herein, the ligand-binding elements from the extracellular components of VEGFR-1 and VEGFR-2 may comprise, or preferably comprise, the second (Ig) domain of VEGFR-1 and the third (Ig) domain of VEGFR-2, respectively, as exemplified by the fusion protein of 229658 or 229657.

As described herein, the function of a growth factor, or the function of a growth factor receptor, may be considered as a function in endothelial cell growth or development, in particular vascular endothelial cell growth or development. Accordingly, a VEGF binding protein may be considered as a vascular endothelial growth factor inhibitor that blocks the growth or proliferation of vascular endothelial cells. Preferably, a therapeutic protein of the present invention may be considered as a (vascular endothelial) growth factor inhibitor that blocks the growth or proliferation of (vascular) endothelial cells in the eye, in particular retinal endotheial cells.

In various embodiments, the fusion polypeptide comprises an immunoglobulin type disease target specific therapeutic protein such as a VEGF specific therapeutic protein. In various preferred embodiments, the immunoglobulin type therapeutic protein is a monoclonal antibody binding to or with specificity to the target such as VEGF. In various preferred embodiments, the antibody binding to or with specificity to the target is a full-length antibody, or a fragment thereof. Such antibody fragments include, but are not limited to, single-chain variable fragments (scFv), single-chain antibodies (scAb), and antigen binding frgments (Fab). The full-length antibody binding to or with specificity to VEGF comprises an Fc domain.

In some embodiments, the fusion protein comprises Aflibercept as VEGF binding protein. In some embodiments, the fusion protein comprises a biosimilar of Aflibercept, for example ABP938, HSA101 (Harmonic), BLA761, M710, SB11, or other VEGF binding proteins.

In some embodiments the fusion protein comprises, in addition to the serum albumin binding protein as described herein, a VEGF specific protein that is an extracellular domain of a receptor or fragments thereof. In some embodiments, the VEGF binding protein is the recombinant fusion protein Aflibercept (SEQ ID NO: 3).

In various embodiments, the fusion protein comprises (or is fused to, or linked to) the Fc region (or Fc domain) of an immunoglobulin (Ig) molecule. Preferably, the Fc region (or Fc domain) is of a human immunoglobulin (Ig) molecule, more preferably the Fc region (or Fc domain) is of a human IgG molecule, and even more preferably the Fc region (or Fc domain) is of a human IgG1 molecule. In various embodiments, the therapeutic protein of the fusion protein of the invention comprises an Fc region of an immunoglobulin, as described elsewhere herein. Accordingly, disclosed herein are fusion proteins comprising a therapeutic protein, which comprises an Fc region of an immunoglobulin, wherein the fusion protein is linked to or fused to an Fc region (or Fc domain) of an Ig molecule as described above, wherein the Fc region (or Fc domain) is different from the Fc region comprised by the therapeutic protein.

In IgG, IgA and IgD antibody isotypes, the Fc region is composed of two identical protein fragments, derived from the second and third constant domains of the antibody's two heavy chains. Accordingly, the Fc region of an IgG consists of two (paired) CH3 domains and two (separated) CH2 domains.

In various embodiments of the fusion protein of the present invention, the serum albumin binding protein is fused to a therapeutic protein comprising an Fc region as described above. The Fc region typically is an Fc region of a human IgG molecule, preferably of a human IgG1 molecule. In preferred embodiments of the present invention, the serum albumin binding protein is fused to such therapeutic proteins via the Fc region of the therapeutic protein. More specifically, the serum albumin binding protein is fused to the CH3 domain of the Fc region. Such preferred embodiments of the present invention are exemplified by SEQ ID NO: 4 and SEQ ID NO: 5.

Various preferred embodiments relate to a fusion protein comprising an amino acid sequence of SEQ ID NO: 4, and wherein the fusion protein exhibits specific binding affinity for VEGF, in particular for human VEGF, more particular for human VEGF-A, in addition to specific binding affinity for serum albumin comprising an amino acid sequence with at least 90 % or 93 % sequence identity to SEQ ID NO: 1. Biosimilars of Aflibercept can be used accordingly.

In some embodiments the fusion protein comprises a) a serum albumin binding protein at least 90 % or at least 93 % identical to SEQ ID NO: 1; and b) a VEGF-antagonist, such as Aflibercept or a biosimilar thereof. A non-limiting example is provided in the amino acid sequence of SEQ ID NO: 4 or SEQ ID NO: 5.

In some embodiments the fusion protein comprises a) a serum albumin binding protein at least 90 % or at least 93 % identical to SEQ ID NO: 1; and b) a VEGF-antagonist, such as Conbercept or a biosimilar thereof.

As described herein, it is preferred in the present invention that variants of a serum albumin binding protein that are described herein by percent % identity to any of the amino acid sequences of SEQ ID NO: 1, exhibit the functional properties (in particular binding affinity and prolongation of half-life) of serum albumin binding proteins of the invention as described elsewhere herein. In particular, such (variants of) serum albumin binding proteins that are described herein by percent % identity to any of the amino acid sequences of SEQ ID NO: 1, exhibit the functional properties (in particular with regard to binding affinity and prolongation of half-life as described elsewhere herein) of serum albumin binding proteins as exemplified by fusion proteins 229658 and 229657, preferably as exemplified by fusion protein 229658. More preferably, (variants of) serum albumin binding proteins that are described herein by percent % identity to any of the amino acid sequences of SEQ ID NO: 1, exhibit the same functional properties (in particular with regard to binding affinity and prolongation of half-life, as described elsewhere herein) as the serum albumin binding proteins of fusion proteins 229658 and 229657, preferably as exemplified by fusion protein 229658.

On other embodiments further components can be included N-terminal and/or C-terminal. Further components may be labels or domains for the purpose of purification or to enhance solubility or for stabilization or for detecting, as known to someone skilled in the art.

Linker. In some embodiments the fusion protein or fusion polypetide as defined above or as defined elsewhere herein can include a linker, for example a peptide linker, between the serum albumin binding protein and the therapeutic protein, or a peptide linker between two serum albumin binding proteins.

The length and composition of a peptide linker may vary between at least one and up to about 50 amino acids. More preferably, the peptide linker has a length of between 1 and 30 amino acids; e.g. 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 amino acids. It is preferred that the amino acid sequence of the peptide linker is not immunogenic to human beings, stable against proteases and optionally does not form a secondary structure. Suitable amino acids for linkers may be selected from amino acids such as glycine, serine, alanine, or proline. A suitable linker of 15 amino acids (glycine and serine) is shown in SEQ ID NO: 6. The linker of SEQ ID NO: 6 is composed of three (glycine / serine) units. The linker of SEQ ID NO: 7 is composed of two (glycine / serine) units. Disclosed herein are suitable linkers that are composed of two or three of these (glycine / serine) units. One embodiment refers to a fusion protein or fusion polypeptide of the invention comprising a serum albumin binding protein of the invention with a binding affinity (K_{D}) of less than 10 nM for serum albumin. Some embodiments refer to fusion protein or fusion polypeptide comprising a serum albumin binding protein of the invention with a binding affinity (K_{D}) of less than 10 nM, preferably less than 5 nM, more preferably less than 1 nM for serum albumin.

One embodiment refers to a fusion protein or fusion polypeptide of the invention comprising a serum albumin binding protein of the invention with a binding affinity (K_{D}) of less than 10 nM for serum albumin. Some embodiments refer to fusion protein or fusion polypeptide comprising a serum albumin binding protein of the invention with a binding affinity (K_{D}) of less than 10 nM, preferably less than 5 nM, more preferably less than 1 nM for human serum albumin, cynomolgus serum albumin, rat serum albumin, or mouse serum albumin.

Further, the fusion proteins bind the VEGF with a measurable binding affinity (K_{D}) of less than 10 nM, less than 5 nM, or less than 1 nM. In some embodiments, the fusion protein binds serum albumin with a binding affinity of less than 10 nM and binds the VEGF with affinity of less than 10 nM, less than 5 nM, or less than 1 nM. In some embodiments, the binding affinities (K_{D}) of the fusion protein for serum albumin and for the target of the therapeutic protein are different. The appropriate methods are known to those skilled in the art or described in the literature. The methods for determining the binding affinities are known per se and can be selected for instance from the following methods known in the art: enzyme-linked immunosorbent assay (ELISA), surface plasmon resonance (SPR), kinetic exclusion analysis (KinExA assay), Bio-layer interferometry (BLI), flow cytometry, fluorescence spectroscopy techniques, isothermal titration calorimetry (ITC), analytical ultracentrifugation, radioimmunoassay (RIA or IRMA), and enhanced chemiluminescence (ECL). Some of the methods are described in the Examples below. Typically, the dissociation constant K_{D} (and, hence, the binding affinity) is determined at 20°C, 25°C, or 30°C, in preferred embodiments at room temperature (25 °C). The lower the K_{D} value, the greater the binding affinity of the biomolecule for its binding partner. The higher the K_{D} value, the more weakly the binding partners bind to each other.

The fusion protein according to 229658 exemplies a particularly preferred embodiments of the present invention. Other preferred embodiments of the present invention are exemplified by 229657.

In some embodiments the half-life of the therapeutic protein is increased by fusion to a serum albumin binding protein as disclosed herein. Concentration can be measured by methods known to some skilled in the art, and include ELISA, mass spectroscopy, western blot, radioimmunoassay, or fluosecent labeling. Methods for pharmocokinetic analysis and determination of half-life and/or mean residence time are known in the art.

In preferred embodiments, the half-life of the therapeutic protein comprising the serum albumin binding protein of the invention, obtained from the concentrations in the eye compartment (e.g. vitreous or retina) over time, is increased at least 1.3fold, or at least 2fold, as compared to the therapeutic protein without the serum albumin binding protein of the invention. In some embodiments, the half-life of the fusion protein comprising a VEGF binding protein and serum albumin binding protein of the invention is at least about 1.3fold longer compared to the VEGF binding protein without serum albumin binding protein.

In some embodiments, the half-life of the fusion protein as described herein is about 1.5 days in the vitreous or 0.8 days in the retina (see EXAMPLE 4).

Preferably, the concentration of the therapeutic protein or of the fusion protein means concentration in the vitreous (vitreous body). More specifically, the concentration of the therapeutic protein or fusion protein means the concentration in the vitreous (vitreous body) after intravitreal administration. In preferred embodiments, the concentration in the eye (in ng/ml) of the fusion protein is at least 5fold higher than the concentration of the therapeutic protein without the albumin binding domain.

Some embodiments relate to the fusion protein as described above for use in the therapy of eye.

In various embodiments, the fusion polypeptide of the invention comprises a VEGF binding protein and a serum albumin specific binding protein, wherein the VEGF binding protein exhibits any of the functional properties described elsewhere herein for a target, e.g. VEGF binding proteins, and wherein the serum albumin specific binding protein exhibits any of the functional properties described elsewhere herein for serum albumin binding proteins. Accordingly, in various emodiments, any of the functional characteristics or properties described herein in relation to a target, e.g. VEGF binding protein may be combined with any of the functional characteristics or properties described herein in relation to a serum albumin binding protein.

In some embodiments, the serum albumin binding protein is a multimer comprising of a plurality of the serum albumin binding protein as defined herein. A multimer may comprise two, three, four, or more serum albumin binding proteins. In one embodiment, the serum albumin binding protein comprises 2, 3, 4, or more serum albumin binding proteins linked to each other, i.e. the serum albumin-binding protein can be a dimer, trimer, or tetramer, etc. In some embodiments, the multimer is a dimer of the serum albumin binding protein as defined above. In some embodiments, the multimeric serum albumin binding protein may comprise at least two modules of proteins that are least 90 % identical to SEQ ID NO: 1. For example, a dimeric serum albumin binding protein may comprise two monomers of SEQ ID NO: 1 linked to each other, preferably by a GS-linker as described above (such as SEQ ID NO: 7 or SEQ ID NO: 6).

Multimers of the binding protein are generated artificially, generally by recombinant DNA technology well-known to a skilled person.

### Use in medicine.

The fusion protein according to the present invention is for example for use in treating any disease or condition which is improved, ameliorated, or inhibited by removal, inhibition, or reduction of VEGF, in particular VEGF-A, and/or PLGF. Conditions and/or diseases for which the fusion protein according to the present invention can be used are preferably angiogenesis-related disease of the eye, more specifically VEGF(A)-related disease or disorders of the eye. Such disease may be characterized by an undesirable plasma leakage or vascular permeability, or undesirable blood vessel growth. Specific eye diseases and disorders of the present invention comprise age related macular degeneration and diabetic retinopathy, without being limited thereto. The novel fusion proteins of the present invention are also particularly useful as an adjuvant to eye surgeries, including glaucoma surgery. Specific eye diseases and disorders of the present invention further comprise, without being limited thereto, intra-ocular tumors, such as for example, uveal melanoma, retinoblastoma.

The novel fudsion proteins of the present invention may preferably be administered by intravitreal administration/delivery.

Various embodiments relate to the fusion protein as defined above comprising a serum albumin binding protein and a VEGF binding protein, such as Aflibercept, for use in the treatment or prevention of neovascular eye diseases and/or angiogenesis. Neovascular eye diseases may be selected from the group of but not limited to neovascular (wet) age-related macular degeneration (AMD), diabetic macular edema (DME), diabetic retinopathy (DR), and macular edema following retinal vein occlusion (RVO).

Some embodiments relate to methods for treating a subject with a disorder that affects the eye including administering to the eye a therapeutically effective amount of the fusion protein as defined herein.

The present invention provides a pharmaceutical composition comprising a fusion polypeptide as described herein, and a pharmaceutical acceptable carrier and/or diluent.

The present invention provides a fusion polypeptide as described herein, or a therapeutical composition comprising a fusion polypeptide as described herein, for use in medicine. The present invention provides a therapeutical composition comprising a fusion polypeptide as described herein, and a therapeutical acceptable carrier and/or diluent.

Various embodiments relate to a composition comprising the fusion protein as defined above comprising a serum albumin binding protein and therapeutic protein such as Aflibercept. The composition optionally may contain further auxiliary agents and excipients known per se.

These include for example but are not limited to stabilizing agents, surface-active agents, salts, buffers, coloring agents etc. The compositions can be in the form of a liquid preparation, a lyophilisate, an aerosol, in the form of powders, granules, in the form of an emulsion or a liposomal preparation.

Various embodiments relate to a pharmaceutical composition for the treatment of diseases affecting the eye comprising a fusion protein as disclosed herein, and a pharmaceutically acceptable carrier and/or diluent. A pharmaceutically acceptable carrier may include solvents, dispersion meida, coatings, antibacterial and antifungal agents, isotonic and absorption delaying agents, and the like that are physiologically compatible. The pharmaceutical composition comprising a fusion protein as defined above can be used for treatment of eye diseases or for the treatment of joint diseases. The pharmaceutical composition may be suitable for intravitreal administration.

The composition for the treatment of eye disorders should be fluid and sterile. In some cases, isotonic agents, polyalcohols, and sodium chloride may be included in the composition. The composition may include an agent which delays absorption, for example, gelatin or aluminum monostearate. The type of pharmaceutical preparation depends on the type of eye disease to be treated, the route of administration, the severity of the disease, the patient to be treated and other factors known to those skilled in the art of medicine.

The compositions contain a therapeutically effective dose of the fusion protein as defined above. The amount of fusion protein to be administered depends on the organism to be treated, the type of disease, the age and weight of the patient and further factors known per se. Depending on the galenic preparation these compositions can be administered by injection, or by other conventionally employed methods of application for the treatment of eye diseases. In various embodiments, the composition is suitable for delivery to the eye by intravitreal, topical ophthalmic, intraretinal, subretinal, suprachoroidal and intracameral delivery.

Various embodiments relate to a method for treating a subject with an eye disease, comprising administering to the eye of the subject a fusion protein or a serum albumin binding protein as defined above. Preferred is a method for treating a subject with an eye disease selected from neovascular eye diseases.

The present invention further provides a method for the prevention and/or treatment of an eye disease in a subject in need thereof, comprising administering to the subject a therapeutically effective amount of a fusion polypeptide or a pharmaceutical composition of the invention as described herein. Typically, the fusion polypeptide is comprised by a pharmaceutical composition. Accordingly, the therapeutic method comprises the administration of a therapeutically effective amount of a pharmaceutical composition comprising a fusion polypeptide of the invention as described herein. Preferably, the subject is a human subject.

In various embodiments of the methods for the prevention and/or treatment disclosed herein, the fusion polypeptide of the invention, or the pharmaceutical composition comprising a fusion polypeptide of the invention, is administered by topical administration or by intraocular aministration. Preferably, the intraocular administration is via intravitreal route (intravitreal administration). The topical administration or intraocular aministration, including the intravitreal route as the preferred intraocular administration, are the administration routes in the treatment or prevention of diseases of the eye, in particular eye diseases as described herein. **Preparation of fusion proteins.** A fusion protein as defined above may be prepared by any of the many conventional and well-known techniques such as plain organic synthetic strategies, solid phase-assisted synthesis techniques, fragment ligation techniques or by commercially available automated synthesizers. On the other hand, they may also be prepared by conventional recombinant techniques alone or in combination with conventional synthetic techniques. Furthermore, they may also be prepared by cell-free *in vitro* transcription/translation or in combination with conventional synthetic techniques.

Various embodiments relate to an isolated polynucleotide encoding a fusion protein as defined above. The invention also encompasses polypeptides encoded by said polynucleotides. The invention further provides an expression vector comprising said polynucleotide, and a host cell comprising said polynucleotide or said expression vector.

Various embodiments relate to a **method for the production** of a fusion protein as defined above comprising culturing of a host cell under suitable conditions in order to obtain said fusion protein and optionally isolating said fusion protein.

Various embodiments relate to a polynucleotide encoding a fusion protein as described above. The invention further provides an expression vector comprising said polynucleotide, and a host cell comprising said isolated polynucleotide or the expression vector.

Various embodiments relate to a method for the production of a fusion protein as described above comprising culturing of a host cell under suitable conditions which allow expression of said fusion protein and optionally isolating said fusion protein.

For example, one or more polynucleotides which encode for fusion protein may be expressed in a suitable host and the produced fusion protein can be isolated. Vectors comprising said polynucleotides are covered herein. A further embodiment relates to a vector comprising said nucleic acid molecule. A vector means any molecule or entity (e.g., nucleic acid, plasmid, bacteriophage or virus) that can be used to transfer protein coding information into a host cell. Furthermore, an isolated cell is disclosed comprising said nucleic acid molecule or said vector. Suitable host cells include prokaryotes or eukaryotes. Various mammalian or insect cell culture systems can also be employed to express recombinant proteins.

An embodiment also relates to a host cell or a non-human host carrying said vector. A host cell is a cell that has been transformed with a nucleic acid sequence and thereby expresses a gene of interest.

Suitable conditions for culturing prokaryotic or eukaryotic host cells are well known to the person skilled in the art. Cultivation of cells and protein expression for the purpose of protein production can be performed at any scale, starting from small volume shaker flasks to large fermenters, applying technologies well-known to any skilled in the art.

One embodiment is directed to a method for the preparation of a binding protein as detailed above, said method comprising the following steps: (a) preparing a nucleic acid encoding a fusion protein as described above; (b) introducing said nucleic acid into an expression vector; (c) introducing said expression vector into a host cell; (d) cultivating the host cell; (e) subjecting the host cell to culturing conditions under which fusion protein is expressed, thereby producing a fusion protein as defined herein; (f) optionally isolating the fusion binding protein produced in step (e); and (g) optionally conjugating the fusion protein with further functional moieties as defined herein.

In general, isolation of purified fusion protein from the cultivation mixture can be performed applying conventional methods and technologies well known in the art, such as centrifugation, precipitation, flocculation, different embodiments of chromatography, filtration, dialysis, concentration and combinations thereof, and others. Chromatographic methods are well-known in the art and comprise without limitation ion exchange chromatography, gel filtration chromatography (size exclusion chromatography), hydrophobic interaction chromatography, or affinity chromatography.

For simplified purification, the fusion protein can be fused to other peptide sequences having an increased affinity to separation materials. Preferably, such fusions are selected that do not have a detrimental effect on the functionality of the fusion protein or can be separated after the purification due to the introduction of specific protease cleavage sites. Such methods are also known to those skilled in the art.

### EXAMPLES

The following Examples are provided for further illustration of the invention. The invention, however, is not limited thereto, and the following Examples merely show the practicability of the invention on the basis of the above description. For complete disclosure of the invention reference is made also to the literature cited in the application which is incorporated completely into the application by reference.

### EXAMPLE 1. Mammalian expression

The N-terminal unit of the fusion proteins were comprised of the second extracellular domain of VEGFR-1 and the third extracellular domain of VEGFR-2, connected to a human IgG1-Fc part. This part is identical to Aflibercept (SEQ ID NO: 3). For fusion protein 229657, Aflibercept was fused at the C-terminus of the Fc to the 15 amino acid GS-linker of SEQ ID NO: 6 and albumin binding domain of SEQ ID NO: 2 (comprising SEQ ID NO: 1). For fusion protein 229658, Aflibercept was fused at the C-terminus of the Fc to a 15 amino acid GS-linker (SEQ ID NO: 6), albumin binding domain of SEQ ID NO: 2 (comprising SEQ ID NO: 1), a 10 amino acid GS-Linker (SEQ ID NO: 7), and albumin binding domain of SEQ ID NO: 2 (comprising SEQ ID NO: 1). Amino acid sequences of the fusion proteins are shown in SEQ ID NO: 4 (229658) and SEQ ID NO: 5 (229657).

Plasmids carrying the genes of the respective proteins were amplified in *E.coli* and purified with standard methods. Mammalian Expi293F cells were transfected with the plasmids and cultivated for several days to allow for protein expression.

### EXAMPLE 2. Purification

Supernatant from the cell culture was clarified and processed on a ProteinA capture column. Protein was eluted and further purified by size exclusion chromatography with a suitable buffer. Protein was then concentrated to around 2 mg/ml. Quality assessment was performed by a number of methods comprising aggregate content by size-exclusion ultra performance liquid chromatography (SE-UPLC) and multi angle light scattering (MALS), as shown in Table **1.** In **Table 1,** SE-UPLC refers to size-exclusion ultra performance liquid chromatography, chromatography, and MALS refers to multi light angle scattering.

**Table 1. Purification of fusion proteins**

| **Parameter** | **Test method** | **Unit** | **Result for fusion proteins** | |
|---|---|---|---|---|
| | | | **229657** | **229658** |
| | | | SEQ ID NO 5 | SEQ ID NO 4 |
| concentration | absorption at 280nm | mg/ml | 3.2 | 1.9 |
| aggregation | SE-UPLC | % | 98 | 100 |
| purity | SDS-PAGE nonreduced | % | 97 | 97 |
| molecular weight | SE-UPLC | kDa | 222.7 | 244.9 |
| molecular weight | MALS | kDa | 136.4 | 156.2 |

### EXAMPLE 3 Binding of fusion proteins to serum albumin and to the VEGF (KD-determination of binding proteins via SPR (Bruker)

A sensor chip (Bruker) was equilibrated with surface plasmon resonance (SPR) running buffer. Surface-exposed carboxylic groups were activated by passing a mixture of EDC and NHS to yield reactive ester groups. 800-1000 RU for the albumins and 600 RU for VEGF were immobilized on separate flow cells, off- ligand was immobilized on another flow cell. Injection of ethanolamine after ligand immobilization deactivates remaining active groups. Upon ligand binding, protein analyte was accumulated on the surface increasing the refractive index. This change in the refractive index was measured in real time and plotted as response or resonance units (RU) versus time. The analytes (SEQ ID NO:4 and 5) were applied to the chip in serial dilutions with a suitable flow rate (µl/min). After each run, the chip surface was regenerated with regeneration buffer and equilibrated with running buffer. As a control-samples were applied to activated/deactivated chip spots. Regeneration and re-equilibration were performed as previously mentioned. Binding studies were carried out by the use of the Bruker SPR-32 system at 25 °C; data evaluation was operated via the Bruker evaluation software, provided by the manufacturer, by the use of the Langmuir 1:1 model (RI=0).

Evaluated dissociation constants (K_{D}) were standardized against off-target and K_{D} values of SEQ ID NO: 4 and SEQ ID NO: 5 for serum albumin of different species (human, cynomolgus, rat) and VEGF were determined, see **Table 2.** HSA = human serum albumin; CSA = cynomolgus serum albumin; RSA = rat serum albumin; MSA = rat serum albumin; kD in nM.

**Table 2: Binding of fusion proteins to serum albumin and VEGF**

| | | **binding vs. (nM)** | | | | |
|---|---|---|---|---|---|---|
| **Fusion protein** | **SEQ ID NO:** | **HSA** | **CSA** | **RSA** | **MSA** | **VEGF** |
| 229657 | 5 | <0.5 | 1.5 | <0.5 | 0.7 | <0.5 |
| 229658 | 4 | 1.2 | 0.9 | <0.5 | <0.5 | <0.5 |

### EXAMPLE 4. Pharmacokinetic study in rats

229658 (30µg) and Aflibercept (24µg) as control were injected intravitreally into rat eyes. Animals were euthanized on days 2, 4, 7, 10, 14, 21 and 28 after injection. Study groups were assigned according to Standard Operating Procedures. All animals were weighed prior to dosing, weekly, and at time of necropsy. A veterinary ophthalmologist performed complete ocular examinations. After necroscopy, the eyes were enucleated, vitreous and retina tissue isolated and the compound concentration in these tissues determined via an ELISA assay. Concentration over time was analyzed to determine the in-vivo half-life of the compounds. The half-life of Aflibercept fused to the albumin binding protein (fusion protein 229658) is improved in the vitreous about 2.5 days compared to Afilbercept, the half-life of fusion protein 229658 is improved in the retina about 1.4 days compared to Afilbercept **(Table 3).** **FIGURE 1** shows that Aflibercept fused to the albumin binding protein has a significantly prolonged half-life compared to Aflibercept without albumin binding protein.

**Table 3: Half-life of fusion protein 229658 in vitreous and retina of rats, compared to Aflibercept. Pharmacokinetic data was fitted to obtain the terminal half-life of each protein in the different compartments of the eye. Half-life in days.**

| | Rat vitreous | Rat retina |
|---|---|---|
| Aflibercept (215631) | 0.70 | 0.60 |
| Fusion protein 229658 | 1.74 | 0.83 |
| improvement xfold | 2.48 | 1.38 |

### EXAMPLE 5. Efficacy study in rabbits

Fusion protein 229658, Aflibercept (referred to as 215631) and vehicle buffer as control were analyzed in 5 sub-groups (each n=12, except vehicle n=6) on day 4, 8, 12, 28 and 42 after injection of 17 pmol protein to about 4-6 months old rabbits *(Oryctolagus cuniculus)* in good health and at 1.3-2.0 kg weight. 20 pMol of VEGF was injected into the vitreous to induce vascular growth 2 days prior to the day of analysis. On the day of analysis, fluorescein was applied systemically and fluorescence angiograms taken from each eye of all rabbits. The formation of blood vessels is not complete and resulting vessels in the retina are leaky, induced by free VEGF. Leakage scores were assigned in a blinded fashion by experienced operators. If sufficient amount of fusion protein is present to neutralize VEGF, no leakage is observed. A score of 0 corresponds to no leakage, a score of 4 to maximal leakage. Therefore, a low score over a long time is indicative of a long presence and half-life of the fusion protein in the eye. Study groups were assigned according to Standard Operating Procedures. All animals were weighed prior to dosing, weekly, and at time of necropsy. Results are shown in **FIGURE 2****.**

### SEQUENCE LISTING

SEQ ID NO: 1 serum albumin binding protein
   LAEAKVLALKELDKYGISNYYKNLINNAKTVEGVKALKEAIVAA
SEQ ID NO: 2 serum albumin binding protein
   LAEAKVLALKELDKYGISNYYKNLINNAKTVEGVKALKEAIVAALP
SEQ ID NO: 3 Aflibercept 
SDTGRPFVEMYSEIPEIIHMTEGRELVIPCRVTSPNITVTLKKFPLDTLIPDGKRIIWDSRKGFII
SNATYKEIGLLTCEATVNGHLYKTNYLTHRQTNTIIDVVLSPSHGIELSVGEKLVLNCTARTEL
NVGIDFNWEYPSSKHQHKKLVNRDLKTQSGSEMKKFLSTLTIDGVTRSDQGLYTCAASSGL
MTKKNSTFVRVHEKDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEYKCKVSNKAL
PAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPG
SEQ ID NO: 4 Fusion protein 229658 
SDTGRPFVEMYSEIPEIIHMTEGRELVIPCRVTSPNITVTLKKFPLDTLIPDGKRIIWDSRKGFII
SNATYKEIGLLTCEATVNGHLYKTNYLTHRQTNTIIDVVLSPSHGIELSVGEKLVLNCTARTEL
NVGIDFNWEYPSSKHQHKKLVNRDLKTQSGSEMKKFLSTLTIDGVTRSDQGLYTCAASSGL
MTKKNSTFVRVHEKDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEYKCKVSNKAL
PAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGGGGG
SGGGGSGGGGSLAEAKVLALKELDKYGISNYYKNLINNAKTVEGVKALKEAIVAALPGGGGS
GGGGSLAEAKVLALKELDKYGISNYYKNLINNAKTVEGVKALKEAIVAALP
SEQ ID NO: 5 Fusion protein 229657 
SDTGRPFVEMYSEIPEIIHMTEGRELVIPCRVTSPNITVTLKKFPLDTLIPDGKRIIWDSRKGFII
SNATYKEIGLLTCEATVNGHLYKTNYLTHRQTNTIIDVVLSPSHGIELSVGEKLVLNCTARTEL
NVGIDFNWEYPSSKHQHKKLVNRDLKTQSGSEMKKFLSTLTIDGVTRSDQGLYTCAASSGL
MTKKNSTFVRVHEKDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEYKCKVSNKAL
PAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGGGGG
SGGGGSGGGGSLAEAKVLALKELDKYGISNYYKNLINNAKTVEGVKALKEAIVAALP
SEQ ID NO: 6 Linker
   GGGGGSGGGGSGGGGS
SEQ ID NO: 7 Linker
   GGGGSGGGGS
SEQ ID NO: 8 Conbercept

GRPFVEMYSEIPEIIHMTEGRELVIPCRVTSPNITVTLKKFPLDTLIPDGKRIIWDSRKGFIISNA
TYKEIGLLTCEATVNGHLYKTNYLTHRQTNTIIDVVLSPSHGIELSVGEKLVLNCTARTELNVG
IDFNWEYPSSKHQHKKLVNRDLKTQSGSEMKKFLSTLTIDGVTRSDQGLYTCAASSGLMTK
KNSTFVRVHEKPFVAFGSGMESLVEATVGERVRIPAKYLGYPPPEIKWYKNGIPLESNHTIKA
GHVLTIMEVSERDTGNYTVILTNPISKEKQSHVVSLVVYVPPGPGDKTHTCPLCPAPELLGG
PSVFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVYTLPPSRDELTK
NQVSLTCLVKGFYPSDIAVEWESNGQPENNYKATPPVLDSDGSFFLYSKLTVDKSRWQQG
NVFSCSVMHEALHNHYTQKSLSLSPGK

## Claims

1. A fusion protein capable of binding to VEGF and serum albumin, comprising:
(i) a VEGF binding protein comprising ligand-binding elements from the extracellular components of VEGF receptor (VEGFR)-1 and/or VEGF receptor (VEGFR)-2; and
(ii) a serum albumin binding protein comprising the amino acid sequence of SEQ ID NO: 1, or an amino acid sequence with at least 90% sequence identity to SEQ ID NO: 1.

2. The fusion protein of claim 1, wherein the ligand-binding elements from the extracellular components of VEGFR-1 and/or VEGFR-2 are fused to the Fc portion of an immunoglobulin, preferably to the Fc portion of an IgG.

3. The fusion protein of claim 1 or 2, wherein the ligand-binding elements from the extracellular components of VEGFR-1 and VEGFR-2 comprise the second (Ig) domain of VEGFR-1 and the third (Ig) domain of VEGFR-2, respectively.

4. The fusion protein of any one of claims 1 to 3, wherein the VEGF binding protein comprises an amino acid sequence with at least 90 %, preferably at least 95 %, sequence identity to SEQ ID NO: 3, and wherein the fusion protein exhibits binding affinity for VEGF, preferably wherein the VEGF binding protein is Aflibercept or a biosimilar of Aflibercept.

5. The fusion protein of any one of claims 1 to 4, wherein the serum albumin binding protein has a binding affinity to human, cynomolgus, rat, and/or mouse serum albumin, of less than 10 nM, preferably of less than 1 nM.

6. The fusion protein of any one of claims 1 to 5, wherein the serum albumin binding protein comprises the amino acid sequence of SEQ ID NO: 2, or an amino acid sequence with at least 90% sequence identity to SEQ ID NO: 2, and/or wherein the VEGF binding protein has a binding affinity to VEGF of less than 10 nM, preferably of less than 1 nM.

7. The fusion protein of any one of claims 1 to 6, wherein the VEGF binding protein and the serum albumin binding protein are linked via a linker, preferably wherein the linker is a peptide linker.

8. The fusion protein of any one of claims 1 to 7, comprising the amino acid sequence of SEQ ID NO: 4 or 5, or an amino acid sequence with at least 90% sequence identity to SEQ ID NO: 4 or 5.

9. The fusion protein of any one of claims 1 to 8, wherein the half-life of the fusion protein is at least 1.5-fold, or at least 2-fold, longer in the vitreous body than the half-life of the VEGF binding protein not fused to serum albumin binding protein.

10. A pharmaceutical composition comprising the fusion protein of any one of claims 1 to 9 and a pharmaceutically acceptable carrier and/or diluent.

11. The fusion protein of any one of claims 1 to 9, or the pharmaceutical composition of claim 10, for use in the treatment of eye diseases, preferably for use in the treatment of neovascular eye diseases.

12. A nucleic acid molecule encoding the fusion protein of any one of claims 1 to 9.

13. A vector comprising the nucleic acid molecule of claim 12.

14. A host cell, or a non-human host, comprising the fusion protein of any one of claims 1 to 9, the nucleic acid molecule of claim 12, and/or the vector of claim 13.

15. A method for the production of the fusion protein of any one of claims 1 to 9, comprising culturing the host cell or the non-human host of claim 14 under conditions suitable to obtain said fusion protein, and optionally isolating the said fusion protein.
